# EUROPÄISCHE PATENTANMELDUNG

(11) **EP 4 498 108 A1**
(43) Veröffentlichungstag der Anmeldung: **29.01.2025**
(21) Anmeldenummer: 23188387.7
(22) Anmeldetag: 28.07.2023
(51) Int. Cl.: G01R 33/36, G01R 33/565, G01R 33/567

(54) **DOPPELRESONANTE MRT LOKALSPULE MIT INTEGRIERTEM PILOT-TON-SIGNAL FREQUENZ-KONVERTER**

(71) Anmelder: Siemens Healthineers AG, 91301 Forchheim (DE)
(72) Erfinder: Bollenbeck, Jan, 91330 Eggolsheim (DE); Speier, Peter, 91056 Erlangen (DE)
(74) Vertreter: Siemens Healthineers Patent Attorneys

(57) **Zusammenfassung**

Die Erfindung betrifft eine Lokalspule für einen Magnetresonanztomographen. Die Lokalspule ist ausgebildet, ein Magnetresonanzsignal und ein Pilottonsignal aufzunehmen und an einen Empfänger des Magnetresonanztomographen zur Auswertung weiterzuleiten. Das Pilottonsignal liegt in einem ersten Frequenzbereich und das Magnetresonanzsignal in einem zweiten Frequenzbereich.

## Beschreibung

Unabhängig vom grammatikalischen Geschlecht eines bestimmten Begriffes sind Personen mit männlicher, weiblicher oder anderer Geschlechteridentität mit umfasst.

Die Erfindung betrifft eine Lokalspule zum Empfang eines Pilottonsignals sowie einen Magnetresonanztomographen mit einer erfindungsgemäßen Lokalspule. Das Magnetresonanzsystem weist einen Empfänger auf, der ausgebildet ist, ein Pilottonsignal und ein Magnetresonanzsignal gleichzeitig zu empfangen und auszuwerten, das ihm von der Lokalspule zugeführt wird.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Magnetresonanzsignal wird dann ausgewertet und eine zwei- oder dreidimensionale bildgebende Darstellung des Untersuchungsobjektes bereitgestellt.

Die Magnetresonanzsignale sind sehr schwach. Um ein ausreichend hohes Signal-zu-Rausch-Verhältnis zu erzielen, ist daher das Signal über eine lange Zeit zu erfassen, in einer oder in wiederholten Messungen. Die Erfassung der Magnetresonanzsignale ist dabei langsam im Vergleich zu unvermeidbaren Bewegungen des Patienten wie Herzschlag oder Atembewegung.

Die Bewegungen verursachen dabei Artefakte in den erzeugten Abbildungen.

Eine Möglichkeit, die bewegten Organe abzubilden besteht dennoch, wenn eine kurze Bilderfassung synchronisiert zu der Bewegung wiederholt ausgeführt wird und über die erfassten Daten mittelt.

Eine Synchronisierung kann mit dedizierten Sensoren wie beispielsweise Atemgurt oder EKG-Elektroden erfolgen.

Um diese zusätzlichen Sensoren zu vermeiden, ist es auch schon aus der Druckschrift US 2015/0320342 A1 bekannt, ein kontinuierliches, monofrequentes Magnetwechselfeld von einer kleinen Leiterschleife ausgehend, zumindest teilweise durch den Körper des Patienten hindurch in die einzelnen Elemente einer Magnetresonanz-Lokalspule zu koppeln.

Da die meisten biologischen Gewebe fast vollständig transparent für Magnetfelder sind, durchdringt das erzeugte Magnetfeld den Körper des Patienten fast unverändert. Die meisten Gewebe sind jedoch (schwach) leitend und daher induziert das kontinuierliche Wellenmagnetfeld Wirbelströme. Diese Wirbelströme erzeugen dann wiederum ein Magnetfeld, das das Anregungsfeld überlagert, was zu Modulationen im empfangenen Magnetfeld in der Empfangsspule führt.

Durch Auswertung dieses Signals kann auf eine Bewegungsphase des Herzens oder der Atmung geschlossen werden.

Es reduziert den Aufwand an Hardware, wenn zum Erfassen der Bewegung ein Signal genutzt wird, das eine Frequenz nahe der Frequenz des Magnetresonanzsignals aufweist und so mit dem gleichen Empfänger, vorzugsweise gleichzeitig, ausgewertet werden kann. Gerade für Herzbewegungen nimmt der Grad der Modulation aber mit der Frequenz stark ab, sodass bei Magnetresonanzsystemen für niedrige statischen Magnetfeldstärken B0, beispielsweise von 0,5T, eine sichere Erkennung des Herzschlages auf diese Weise kaum mehr möglich ist.

Es ist daher eine Aufgabe der vorliegenden Erfindung, das Erfassen von Bewegungen des Patienten bei Niederfeldsystemen besser und zuverlässiger zu machen.

Die Aufgabe wird durch eine erfindungsgemäße Lokalspule nach Anspruch 1 und einen erfindungsgemäßen Magnetresonanztomographen nach Anspruch 9 gelöst.

Die erfindungsgemäße Lokalspule ist zur Verwendung mit einem Magnetresonanztomographen vorgesehen, um Magnetresonanzsignale eines Patienten oder Objektes in einem statischen Magnetfeld B0 des Magnetresonanztomographen aufzunehmen. Die Frequenz des Magnetresonanzsignals ist definiert durch die Magnetfeldstärke B0 und ein magnetisches Moment der zu erfassenden Kernspins und wird als Larmorfrequenz bezeichnet.

Die erfindungsgemäße Lokalspule ist insbesondere ausgebildet, ein Magnetresonanzsignal und ein Pilottonsignal aufzunehmen und an einen Empfänger des Magnetresonanztomographen zur Auswertung weiterzuleiten. Mit Aufnehmen ist dabei insbesondere das Wandeln des magnetischen bzw. elektromagnetischen hochfrequenten Wechselfeldes der Kernspins und des Pilottonsignals durch eine Antenne bzw. Induktionsschleife in ein elektrisches Signal bezeichnet. Mit Pilotton wird ein magnetisches bzw. elektromagnetisches Hochfrequenzsignal bezeichnet, dass von einem Pilottonsender, vorzugsweise integriert in die Lokalspule, emittiert wird und mit dem Patienten wechselwirkt, sodass durch dessen Bewegungen wie Atembewegung oder Herzschlag eine Modulation des Pilottons zu einem Pilottonsignal erfolgt. Das Aufnehmen kann auch weitere Signalverarbeitungsschritte wie Verstärken oder Filtern umfassen.

Der Pilotton und damit das Pilottonsignal, das von der Antenne bzw. Induktionsschleife aufgenommen wird, liegen in einem ersten Frequenzbereich, wobei mit Frequenzbereich ein Spektralbereich bezeichnet ist, der die Frequenz des Pilottons und zumindest durch die Modulation verursachte Seitenbänder umfasst. Der erste Frequenzbereich kann eine Bandbreite von mehr als 10 Hz, 100 Hz, 1 kHz, 10 kHz oder auch 100 kHz umfassen.

Das von der Antenne bzw. Induktionsschleife aufgenommene Magnetresonanzsignal liegt in einem zweiten Frequenzbereich, der die bereits definierte Larmorfrequenz des Magnetresonanztomographen umfasst, mit dem die Lokalspule eingesetzt wird. Die Bandbreite des zweiten Frequenzbereichs wird im Wesentlichen durch die Bandbreite der Magnetresonanzsignale vorgegeben, die wiederum von zu erfassenden Schichtdicken und Magnetfeldstärken von Gradienten zur Ortskodierung abhängt. Eine Bandbreite des zweiten Frequenzbereichs kann mehr als 100 kHz, 50 kHz, 1 MHz oder 5MHz umfassen. Nachfolgend wird auch das bearbeitete Magnetresonanzsignal, gegebenenfalls auch in der Frequenz umgesetzte Magnetresonanzsignal als solches bezeichnet.

Der erste Frequenzbereich und der zweite Frequenzbereich sind dabei disjunkt. Ein Signal, das in dem ersten Frequenzbereich ist, ist nicht Teil des zweiten Frequenzbereichs und umgekehrt. Wie nachfolgend zu den Unteransprüchen dargelegt, ist es insbesondere auch denkbar, dass eine Frequenz in dem ersten Frequenzbereich ein ganzzahliges Vielfaches einer Frequenz im zweiten Frequenzbereich ist. Die Frequenzbereiche weisen einen Frequenzabstand auf, der wesentlich im Vergleich zu z.B. einer Mittenfrequenz oder einer Bandbreite eines der Frequenzbereiche ist, beispielsweise größer als ein ganzzahliges Vielfaches mit n>1 davon.

Die erfindungsgemäße Lokalspule weist einen Frequenzumsetzer auf. Ein Frequenzumsetzer ist eine Vorrichtung, die ein Signal von einer Frequenz auf eine andere umsetzt. Ein Frequenzumsetzer kann beispielsweise durch einen Mischer realisiert sein, der ein erstes Signal mit einer ersten Frequenz durch Mischen, z.B. Multiplizieren oder einer anderen nichtlinearen Operation auf eine oder mehrere andere Frequenzen umsetzt.

Gemäß der Erfindung setzt der Frequenzumsetzer der Lokalspule das aufgenommene Pilottonsignal in einen gemeinsamen Frequenzbereich mit dem Magnetresonanzsignal um.

Dazu ist es zum einen denkbar, dass ein erster Frequenzumsetzer das Pilottonsignal in den Frequenzbereich umsetzt, in den auch das von den Kernspins emittierte Magnetresonanzsignal fällt, also der gemeinsame Frequenzbereich der zweite Frequenzbereich ist.

Es ist aber auch denkbar, dass der erste Frequenzumsetzer das Pilottonsignal in einen gemeinsamen Frequenzbereich umsetzt, der disjunkt zu dem ersten Frequenzbereich und dem zweiten Frequenzbereich ist. Das von der Antenne erfasste Magnetresonanzsignal wird von einem zweiten Frequenzumsetzer oder auch von dem ersten Frequenzumsetzer in den gemeinsamen Frequenzbereich umgesetzt. Dies kann beispielsweise eine Zwischenfrequenz sein, die Rückkopplungen auf das Eingangssignal verhindert, die Dämpfung bei der Übertragung zum Magnetresonanztomographen reduziert und/oder einem Frequenzmultiplex dient.

Mit anderen Worten, das Pilottonsignal wird von dem ersten Frequenzbereich in einen gemeinsamen Frequenzbereich mit dem Magnetresonanzsignals umgesetzt, sodass das umgesetzte Pilottonsignal anschließend von einem Empfänger für das Magnetresonanzsignal verarbeitet und ausgewertet werden kann.

Vorzugsweise erfolgt die Frequenzumsetzung derart, dass das Magnetresonanzsignal und das umgesetzte Pilottonsignal in dem zweiten Frequenzbereich nicht überlappen, sondern nebeneinander liegen. Dies lässt sich beispielsweise durch eine geeignete Auswahl der Frequenz eines Misch- bzw. Oszillatorsignals erreichen.

Auf vorteilhafte Weise ermöglicht der Frequenzumsetzer der erfindungsgemäßen Lokalspule, dass das Pilottonsignal eine wesentlich höhere Frequenz aufweist als das Magnetresonanzsignal und dennoch von einem Empfänger für das Magnetresonanzsignal verarbeitet werden kann. Insbesondere bei Magnetresonanztomographen mit niedrigem statischem Magnetfeld kann so kostengünstig eine Pilottonlösung mit hoher Bewegungssensitivität bereitgestellt werden.

Weitere vorteilhafte Ausführungsformen sind zu den Unteransprüchen ausgeführt.

In einer denkbaren Ausführungsform weist die erfindungsgemäße Lokalspule einen Eingang für ein Oszillatorsignal auf. Als Oszillatorsignal wird ein Signal verstanden, das eine Referenzfrequenz vorgibt, die vorzugsweise hochkonstant ist. Das Oszillatorsignal kann beispielsweise ein analoges Sinussignal sein, dass elektrisch über eine Strom- oder Spannungsmodulation übertragen wird. Denkbar ist auch die Modulation einer Trägerwelle, beispielsweise für eine drahtlose Übertragung über Radiowelle, optisch oder per Induktion durch ein moduliertes Magnetfeld. Der Eingang kann entsprechend ein elektrischer oder optischer Eingang, eine Antenne oder eine Induktionsschleife sein. Möglich ist auch eine digitale Übertragung mittels Codierung auf einem entsprechenden Trägersignal.

Die erfindungsgemäße Lokalspule ist dabei ausgebildet, aus dem Oszillatorsignal einen Pilotton zum Aussenden bzw. Koppeln zu erzeugen. Denkbar ist es beispielsweise, dass das Oszillatorsignal mittels eines Frequenzumsetzers auf eine andere Frequenz umgesetzt oder vervielfacht wird. Es ist aber auch denkbar, dass das Oszillatorsignal zusätzlich oder lediglich verstärkt und/oder gefiltert wird.

Die erfindungsgemäße Lokalspule ist weiterhin ausgebildet, ein Mischersignal zum Frequenzumsetzen des Pilottonsignals mit dem Frequenzumsetzer aus dem Oszillatorsignal zu erzeugen bzw. aus diesem abzuleiten. Wird der Pilotton durch eine Frequenzvervielfachung erzeugt, so kann das Mischersignal ebenfalls durch eine Frequenzvervielfachung aus dem Oszillatorsignal erzeugt werden. Es ist aber auch denkbar, dass das Oszillatorsignal eine hohe Frequenz aufweist, beispielsweise höher als das Mischersignal und/oder der Pilotton, sodass Mischersignal und/oder Pilotton durch Frequenzteilung erzeugt werden. Einige besonders vorteilhafte Konstellationen sind nachfolgend näher erläutert. Als Frequenzvervielfachung wird dabei eine Vervielfachung mit ganzzahligen Faktoren angesehen, wie sie z.B. durch Oberwellen bzw. Harmonischen erzielt wird, aber auch um rationale Faktoren, wie sie beispielsweise durch Synthesizer bzw. PLL realisiert werden können.

Auf vorteilhafte Weise führt die Erzeugung sowohl von Pilotton als auch Mischersignal aus einem gemeinsamen, zugeführten Oszillatorsignal zu einer hohen Frequenzstabilität und Phasensynchronität des Pilottonsignals und damit auch zur Vermeidung von Störungen der Magnetresonanzabbildung.

In einer möglichen Ausführungsform der erfindungsgemäßen Lokalspule weist die Lokalspule einen ersten Frequenzvervielfacher mit einem ersten Vervielfachungsfaktor m zum Erzeugen des Pilottons aus dem Oszillatorsignal und einen zweiten Frequenzvervielfacher mit einem zweiten Vervielfachungsfaktor n zum Erzeugen des Mischersignals aus dem Oszillatorsignal auf. Mit anderen Worten, die Frequenz des Pilottons ist ein m-faches der Frequenz des Oszillatorsignals und die Frequenz des Mischersignals ist ein n-faches der Frequenz des Oszillatorsignals.

Auf vorteilhafte Weise werden aus einem einzigen Oszillatorsignal sowohl der Pilotton als auch das Mischersignal zum Heruntermischen des Pilottonsignals erzeugt, was das Frequenzschema vereinfacht und mögliche Störquellen für das Magnetresonanzsignal reduziert.

In einer bevorzugten Ausführungsform der erfindungsgemäßen Lokalspule unterscheiden sich der erste Vervielfachungsfaktor m und der zweite Vervielfachungsfaktor n um die Zahl 1. Mit anderen Worten, der Betrag der Differenz (n-m) ist gleich 1. Vorzugsweise sind dabei m und n natürliche Zahlen größer 1, es ist aber auch denkbar, dass eine von beiden den Wert 1 hat.

Auf vorteilhafte Weise führt eine Frequenzvervielfachung mit den genannten Zahlenverhältnissen dazu, dass mindestens eines der Mischprodukte wieder stabil die Frequenz des Oszillatorsignals aufweist, was eine Vermeidung von Bildartefakten durch Störsignale in Form von Mischprodukten mit weiteren Frequenzwerten reduziert und eine Verarbeitung durch einen Empfänger für das Magnetresonanzsignal ermöglicht.

In einer denkbaren Ausführungsform der erfindungsgemäßen Lokalspule weist das Mischersignal die gleiche Frequenz wie das Oszillatorsignal auf. Mit anderen Worten, das Oszillatorsignal wird nicht in der Frequenz durch Teilen, Vervielfachen oder Mischen verändert, bevor es als Mischersignal genutzt wird. Der Frequenzumsetzer, dem das Mischersignal zugeführt wird, ist ein Schalt-Mischer. Unter Schaltmischer ist dabei ein Mischer zu verstehen, der nicht eine analoge Multiplikation der beiden zu mischenden Signale vornimmt, sondern gesteuert durch die Mischerfrequenz wird die Polarität der zu mischenden Frequenz umgeschaltet. Denkbar ist statt einer Polaritätsumschaltung auch eine Ein- und Ausschalten. Das Mischersignal ist dabei vorzugsweise ein symmetrisches Rechtecksignal, d.h. mit gleich langen Periodendauern für beide Zustände.

Auf vorteilhafte Weise wirkt ein Schaltmischer aufgrund der Fourieräquivalenz wie ein analoger Mischer mit einem Mischersignal, das aus einer Fourierreihe mit ungeraden Vielfachen der Grundfrequenz als Komponenten besteht. So kann auf einen separaten Frequenzumsetzer zur Erzeugung des Mischersignals aus dem Oszillatorsignal verzichtet werden. Die aufgrund der geringeren Amplitude der Mischersignal-Harmonischen reduzierte Mischer-Umsetzverstärkung (Mixer Conversion Gain) für das Pilottonsignal kann durch eine entsprechende Verstärkung bzw. Frequenzabhängigkeit der nachfolgenden Signalverarbeitung kompensiert werden.

In einer möglichen Ausführungsform der erfindungsgemäßen Lokalspule weist die Lokalspule eine Antennenspule zum Empfang des Pilottonsignals und des Magnetresonanzsignals auf. Mit anderen Worten, die Lokalspule ist ausgelegt, mit einer Antennenspule gleichzeitig das Pilottonsignal und das Magnetresonanzsignal mit Signalpegeln aufzunehmen, die eine Auswertung in Form einer Bildgebung und einer Bewegungserkennung erlauben. Die Antennenspule weist dazu gleichzeitig eine Resonanz in dem ersten Frequenzbereich und dem zweiten Frequenzbereich auf, also für das Pilottonsignal und das Magnetresonanzsignal.

Auf vorteilhafte Weise ist so auch für Magnetresonanzsignale und Pilottonsignale mit wesentlich unterschiedlichen Frequenzen keine separaten Antennenspulen erforderlich, was entsprechend Platz und auch Aufwand für das Detuning reduziert.

In einer denkbaren Ausführungsform der erfindungsgemäßen Lokalspule weist die Lokalspule ein Dämpfungsglied auf. Als Dämpfungsglied wird ein elektronisches Bauelement oder eine Schaltung angesehen, die einen Signaleingang und einen Signalausgang aufweist, wobei das Dämpfungsglied ein am Signaleingang angelegtes Signal auf zumindest einer vorbestimmten Frequenz um einen vorbestimmten Dämpfungsfaktor im Pegel reduziert am Signalausgang ausgibt. Das Dämpfungsglied ist ausgebildet, das Pilottonsignal auf einen Pegel vergleichbar oder niedriger zu dem zu empfangenden Magnetresonanzsignal zu dämpfen, bevor es an den Magnetresonanztomographen zur gemeinsamen Auswertung mit dem Magnetresonanzsignal weitergeleitet wird. Unter vergleichbar wird verstanden, dass der Pegel des Pilottonsignals nach dem Dämpfungsglied gleich oder unterhalb eines Pegels des empfangenen Magnetresonanzsignals liegt, insbesondere einem maximal zu empfangenden Magnetresonanzsignal, das durch eine Obergrenze des linearen Verstärkungsbereichs eines Eingangsverstärkers des Empfängers des Magnetresonanztomographen definiert ist. Die Dämpfung des empfangenen Pilottonsignals gegenüber dem empfangenen Magnetresonanzsignal durch das Dämpfungsglied ist vorzugsweise größer als 3 dB, 6dB oder 9 dB. Vorzugsweise ist die Dämpfung aber auch nicht größer als 12 dB, 18 dB oder 24 dB.

Das Dämpfungsglied für das Pilottonsignal ist dabei vorzugsweise vor der Frequenzumsetzung im Signalpfad des Pilottonsignal angeordnet, kann aber auch nach der Frequenzumsetzung im Signalpfad des Pilottonsignal angeordnet sein oder auch Teil der Frequenzumsetzung sein.

Bei einer Ausführungsform der erfindungsgemäßen Lokalspule mit einer Mehrzahl an Antennenspulen in einer Antennenmatrix ist es dabei denkbar, dass aufgrund der unterschiedlichen Abstände der Antennenspulen zu einer in der Lokalspule integrierten Sendeantenne bzw. Induktionsschleife für den Pilotton die Dämpfung für das Dämpfungsglied für den Pilotton unterschiedlich für die einzelnen den Antennenspulen zugeordneten Signalpfaden ausgelegt ist. Vorzugsweise würde die Dämpfung mit zunehmendem Abstand der Antennenspule zu der Sendeantenne wegen der geringeren zu erwartenden Pegel des Pilottonsignals abnehmen. Es ist auch denkbar, dass das Dämpfungsglied von einer Steuerung eines Magnetresonanztomographen einstellbar ist, um beispielsweise unterschiedliche Anordnungen bei eine flexiblen Lokalspulenmatrix berücksichtigen zu können.

Auf vorteilhafte Weise ermöglicht das Dämpfungsglied in Verbindung mit dem großen Frequenzabstand des Pilottonsignals zur Larmorfrequenz, dass nur das Pilottonsignal im Vergleich zu dem Magnetresonanzsignal gedämpft wird. Damit kann das Pilottonsignal mit wesentlich höherem Pegel ausgesendet werden und das SNR für die Auswertung des Pilottonsignals verbessert werden. So erfolgt die Erkennung von Bewegungen wie beispielsweise Herzschlag oder Atembewegung stabiler und zuverlässiger.

In einer möglichen Ausführungsform der erfindungsgemäßen Lokalspule ist die Lokalspule ausgebildet, den Pilotton mit einem höheren Pegel als das zu empfangende Magnetresonanzsignal auszusenden. Mit anderen Worten, wegen des Frequenzabstandes von Larmorfrequenz und dem Pilotton kann dessen Pegel auf vorteilhafte Weise größer sein als der eines zu empfangenden Magnetresonanzsignals, ohne dessen Empfang zu stören.

Damit kann das SNR des Pilottonsignals verbessert und die Bewegungserkennung mittels Pilotton verbessert werden, insbesondere stabiler und zuverlässiger erfolgen.

In einer denkbaren Ausführungsform des erfindungsgemäßen Magnetresonanztomographen weist der Magnetresonanztomograph eine erfindungsgemäße Lokalspule auf, weiterhin einen Oszillator und einen Empfänger. Der Magnetresonanztomograph ist ausgebildet, mit dem Oszillator der Lokalspule das Oszillatorsignal bereitzustellen. Dies betrifft insbesondere die Wahl der Frequenz und deren Stabilität. Das Oszillatorsignal und das bzw. die davon abgeleiteten Mischersignale müssen zum einen geeignet sein, die Magnetresonanzsignale und/oder Pilottonsignale auf die gewünschten Frequenzen bzw. Zwischenfrequenzen umzusetzen und gleichzeitig so gewählt werden, dass entsprechende Mischprodukte und Oberwellen nicht mit den empfangenen Magnetresonanzsignalen und Zwischenfrequenzen interferieren. Darüber hinaus ist der Empfänger ausgelegt, das Pilottonsignal und das Magnetresonanzsignal, das er von der erfindungsgemäßen Lokalspule erhält, für eine Bilderfassung und eine Bewegungserkennung auszuwerten. Insbesondere sind das Oszillatorsignal und die Frequenzumsetzer und Mischer so ausgelegt, dass sie in einen gemeinsamen Frequenzbereich fallen, der von dem Empfänger ausgewertet werden kann. Ein detailliertes Beispiel für ein geeignetes Frequenzschema ist nachfolgend zu den Figuren ausgeführt.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung eines erfindungsgemäßen Magnetresonanzsystems;
- Fig. 2: eine schematische Darstellung einer beispielhaften Ausführungsform einer erfindungsgemäßen Lokalspule;
- Fig. 3: eine schematische Darstellung von Teilen einer beispielhaften Ausführungsform einer erfindungsgemäßen Lokalspule;
- Fig. 4: eine schematische Darstellung einer beispielhaften Ausführungsform einer erfindungsgemäßen Lokalspule.

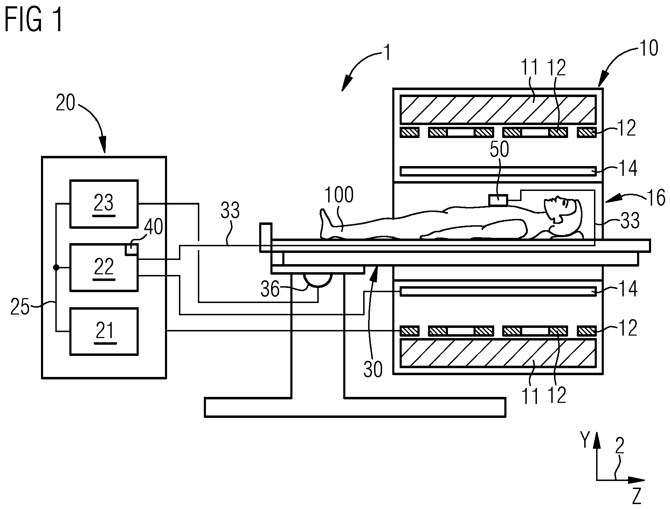

Fig. 1 zeigt eine schematische Darstellung einer Ausführungsform eines erfindungsgemäßen Magnetresonanzsystems 1.

Die Magneteinheit 10 weist einen Feldmagneten 11 auf, der ein statisches Magnetfeld B0 zur Ausrichtung von Kernspins von Proben bzw. des Patienten 100 in einem Aufnahmebereich erzeugt. Der Aufnahmebereich zeichnet sich durch ein äußerst homogenes statisches Magnetfeld B0 aus, wobei die Homogenität insbesondere die Magnetfeldstärke bzw. die Orientierung und den Betrag betrifft. Der Aufnahmebereich ist nahezu kugelförmig und in einem Patiententunnel 16 angeordnet, der sich in einer Längsrichtung 2 durch die Magneteinheit 10 erstreckt. Eine Patientenliege 30 ist in dem Patiententunnel 16 von der Verfahreinheit 36 bewegbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 100 emittierte Resonanzsignale zu empfangen und über eine Signalleitung abzugeben.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die Anregungspulse können über die Körperspule 14 oder auch über eine lokale Sendeantenne in den Patienten 100 abgestrahlt werden.

Die Hochfrequenzeinheit 22 weist auch einen Empfänger 40 zum Empfang bzw. Aufbereitung eines Magnetresonanzsignals aus dem Patienten 100 auf, das von der Lokalspule 50 aufgenommen wird und über eine Signalverbindung zu dem Empfänger 40 überträgt. Der Empfänger 40 ist darüber hinaus auch ausgebildet, einen Pilotton zu empfangen und auszuwerten.

Die Hochfrequenzeinheit 22 weist in einer Ausführungsform auch einen Pilottonsender 70 auf, der einen Pilotton an eine Pilotton-Sendeantenne bzw. Koppelschleife, möglichweise auch in der Lokalspule 50, zum Auskoppeln über eine Signalverbindung überträgt. Denkbar ist aber auch ein separater Pilottonsender 70, der unabhängig vom Magnetresonanzsystem 1 agiert.

Eine Steuerung 23 kommuniziert über einen Signalbus 25 mit der Gradientensteuerung 21 und der Hochfrequenzeinheit 22.

Auf dem Patienten 100 ist eine Lokalspule 50 angeordnet, die über eine Anschlussleitung 33 mit der Hochfrequenzeinheit 22 und deren Empfänger verbunden ist.

Vorzugsweise weist die Lokalspule 50 weiterhin eine Pilotton-Sendeantenne bzw. Koppelschleife auf, mit der ein Pilotton in den Körper des Patienten 100 ausgesendet bzw. induziert werden kann. Vorzugsweise wird der Pilotton in Form eines magnetischen Wechselfeldes von der Koppelschleife erzeugt, das den Körper des Patienten 100 zumindest teilweise durchdringt und in die Antennenspulen 51 des Magnetresonanztomographen 1 koppelt.

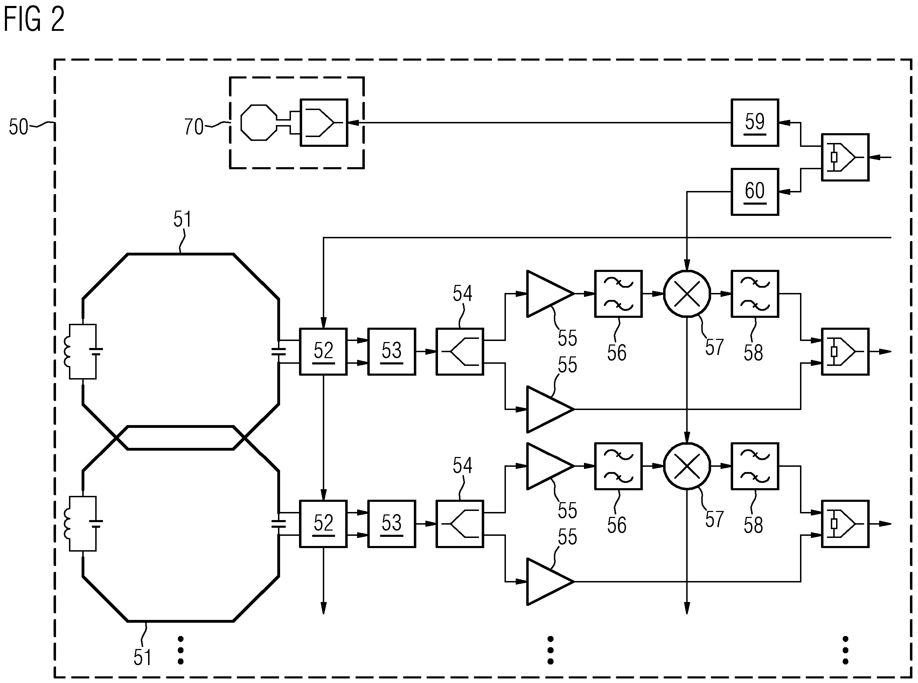

Fig. 2 zeigt eine schematische Darstellung einer beispielhaften Ausführungsform einer erfindungsgemäßen Lokalspule 50.

Die Lokalspule 50 weist eine Mehrzahl an Antennenspulen 51 auf. Die Antennenspulen 51 dieser Ausführungsform sind sowohl zum Empfang des Magnetresonanzsignals und des Pilottonsignals mit wesentlich unterschiedlichen Frequenzen ausgebildet. Um auf beiden Frequenzen eine möglichst hohe Sensitivität zu erzielen, sind die Antennenspulen 51 doppel-resonant auf diesen Frequenzen. Dies kann beispielsweise, wie in der Fig. 2 dargestellt, durch einen Parallel-Resonanzkreis erreicht werden, der in die Schleife der Antennenspule 51 geschaltet ist.

Die Antennenspulen 51 sind weiterhin mit einer Verstimmvorrichtung 52 verbunden, die die Antennenspule 51 verstimmt, um sie während des Anregungspulses zu schützen.

Ein nachfolgender Anpassschaltkreis 53 (Matching-Circuit) sorgt für eine Impedanzanpassung der Antennenspule 51 an die nachfolgende Signalverarbeitung. Der Anpassschaltkreis 53 kann dabei wie in Fig. 2 die Funktion eines Un-Balancers aufweisen, den Übergang von dem symmetrischen Antennensignal zum symmetrischen Verstärkereingang.

In einem Diplexer 54 werden das Pilottonsignal und das Magnetresonanzsignal aufgrund ihrer unterschiedlichen Frequenzen getrennt und dann in Vorverstärkern bzw. LNA 55 verstärkt. In einem ersten Filter 56 wird anschließend das Pilottonsignal gefiltert, um bei dem anschließenden Heruntermischen in dem Mischer 57 unerwünschte Frequenzanteile zu vermeiden. Anschließend wird das Mischprodukt in einem zweiten Filter 58 auf das Frequenzband der Magnetresonanzsignale beschränkt, sodass es gemeinsam mit dem Magnetresonanzsignal übertragen und von dem Empfänger 40 ausgewertet werden kann. Die Erzeugung der Mischfrequenzen und ein beispielhaftes Frequenzschema werden nachfolgend dargestellt.

Die Reihenfolge der einzelnen Verarbeitungsschritte kann teilweise auch vertauscht werden, insbesondere für lineare Verarbeitungsschritte. Auch ist es denkbar Funktionen zu kombinieren, beispielsweise Anpassung und Filtern.

Insbesondere ist es auch denkbar, dass in dem separaten Signalpfad des Pilottonsignals ein Dämpfungsglied vorgesehen ist, mit dem ein Pegel des Pilottonsignals auf einen Pegel abgesenkt wird, der kleiner oder gleich einem Pegel des empfangenen Magnetresonanzsignals ist. Das Dämpfungsglied kann prinzipiell im gesamten Signalpfad des Pilottonsignals angeordnet sein, bevor es mit dem Magnetresonanzsignal zur weiteren Verwertung zusammengeführt wird. Vorzugsweise ist das Dämpfungsglied vor der Frequenzumsetzung des Pilottonsignals in den Frequenzbereich des Magnetresonanzsignals angeordnet, sodass keine hohen Signalpegel im Frequenzbereich des Magnetresonanzsignals auftreten, die stören könnten. Denkbar ist auch, dass das Dämpfungsglied integraler Bestandteil der bereits beschriebenen Funktionsgruppen, beispielsweise LNA 55 oder erster Filter 56 im Signalpfad ist.

Die Lokalspule 50 der Fig. 2 weist weiterhin einen Pilottonsender 70 auf. Die Lokalspule 50 erhält dazu über einen Signaleingang ein Oszillatorsignal von dem Magnetresonanztomographen 1, von dem der Pilotton und die Mischfrequenz abgeleitet werden. Der Pilotton wird von dem Pilottonsender 70 von dem Oszillatorsignal abgeleitet bzw. aus diesem erzeugt und über die Pilotton-Sendeantenne bzw. Koppelschleife als ein magnetisches Wechselfeld in die Umgebung ausgekoppelt.

Im Folgenden sei ein beispielhaftes Frequenzschema ausgeführt. Bestimmendes Element für das Frequenzschema ist der Feldmagnet 10, der mit der Art der zu detektierenden Kernspins die Frequenz des Magnetresonanzsignal bestimmt, die sogenannte Larmorfrequenz. Für die Beispielrechnung sei hier für ein Niederfeldsystem ein Wert von 23,6 MHz angenommen. Das Oszillatorsignal weist eine naheliegende Frequenz auf, die aber nicht mehr im Frequenzbereich bzw. Bandbreite des Magnetresonanzsignals liegt und die Bilderfassung nicht stört. Als Beispielwert sei hier eine Frequenz von 22,5 MHz angenommen.

In einem ersten Frequenzvervielfacher 59 wird daraus das Pilottonsignal erzeugt, das über eine Induktionsspule in den Patienten ausgekoppelt wird. Ein Vervielfachungsfaktor kann beispielsweise 5 sein, sodass das Pilottonsignal eine Frequenz von 112,5 MHz aufweist. Die Mischfrequenz wird durch einen zweiten Frequenzvervielfacher 60 aus dem Oszillatorsignal erzeugt, wobei sich der Faktor unterscheidet. Beispielsweise kann der Faktor hier 4 sein, sodass die Mischfrequenz 4*22,5 MHz = 90 MHz ist.

Vervielfachungen um den Faktor 4 kann beispielsweise mit einem Vollweg-Gleichrichter und anschließender Filterung der Harmonischen erzielt werden, währen ungeradzahlige Harmonische durch Begrenzung / Rechteckformung generiert werden können.

Werden das Pilottonsignal und das Mischsignal in dem Mischer 57 gemischt, so ergibt sich unter anderem ein Mischprodukt mit einer Frequenz, die der Differenz der Frequenz des Mischsignals und des Pilottonsignals entspricht, also mit (5-4)*22,5 MHz = 22,5 MHz wieder der Frequenz des Oszillatorsignals. Denkbar ist auch, dass der Vervielfachungsfaktor der Frequenz des Mischsignals um eines höher ist. Möglich sind aber auch andere Schemata, beispielsweise mit einer kleineren Frequenz des Oszillatorsignals, die der halben Frequenz gleich 11,25 MHz entspricht. Es können dann Vervielfachungsfaktoren mit einer Differenz von 2 ausgewählt werden, die die Verwendung einfacher Frequenzvervielfacher mit geradzahligen Harmonischen für beide Signale erlaubt.

Das Signal-zu-Rauschverhältnis (SNR) des MR-Signals darf durch die Kombination mit dem PT-Empfangssignal nicht merklich verringern werden, z.B. um weniger als 50mdB. Es ist daher durch geeignete Auslegung des Pilottonsignal-Pegels von der Antennenspule 51 bis hin zum Zusammenführen des Magnetresonanzsignals mit dem Pilottonsignals zur Übertragung an den Magnetresonanztomographen dafür zu sorgen, dass beim Zusammenführen der Rauschflur des PT-Empfangssignals um mindestens 20dB unterhalb des Rauschflurs des MR-Empfangssignals bleibt. Dies lässt sich durch entsprechende Dimensionierung der Vorverstärker und/oder durch unsymmetrische Auslegung des Zusammenführens erreichen, z.B. der Widerstandswerte bei einem Summierglied, das hier die Funktion des Dämpfungsgliedes übernimmt. Die hierdurch im Vergleich zum Magnetresonanzsignal-Empfangspfad erhöhte Rauschzahl im Pilottonsignal-Empfangspfad kann durch eine entsprechende höhere Pilottonamplitude kompensiert werden.

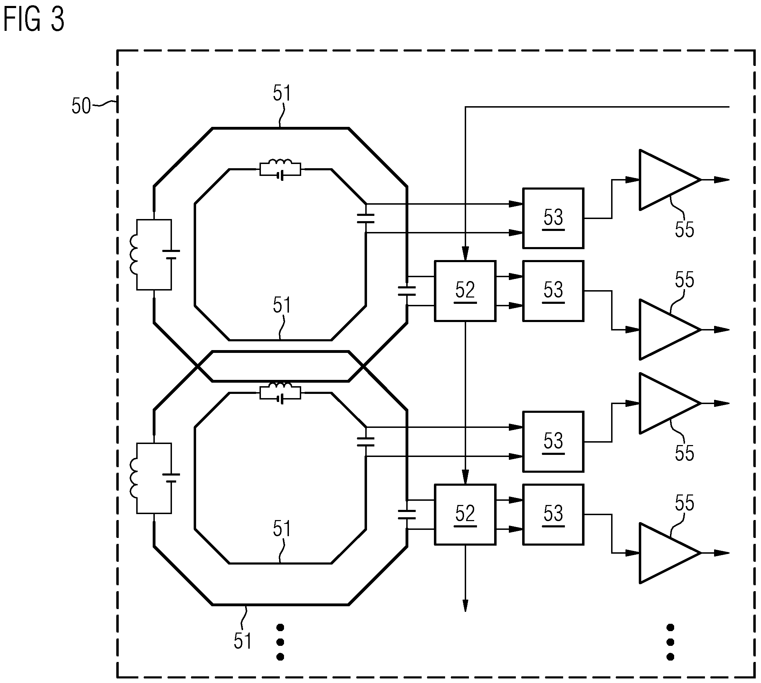

Fig. 3 zeigt eine schematische Darstellung von Teilen einer beispielhaften Ausführungsform einer erfindungsgemäßen Lokalspule 50. Die Lokalspule 50 der Fig. 3 unterschiedet sich dadurch von der Ausführungsform der Fig. 2, dass keine doppelresonante Antennenspule 51 gemeinsam für den Empfang des Pilottonsignals und des Magnetresonanzsignals genutzt wird, sondern jeweils separate Antennenspulen 51. Es sind nur die Teile in Fig. 3 dargestellt, die sich von der Fig. 2 unterscheiden.

In der Fig. 3 sind insgesamt vier Antennenspulen 51 dargestellt, jeweils Paare aus einer Antennenspule 51 für das Pilottonsignal und einer Antennenspule 51 für das Magnetresonanzsignal, deren Ausgangssignale gemeinsam einem Empfänger 40 des Magnetresonanztomographen 1 zugeführt werden. Die Antennenspulen 51 der Paare sind in Fig. 3 konzentrisch angeordnet, dies ist aber nicht zwingend notwendig, reduziert aber den Platzbedarf. Auch kann eine Antennenspule 51 für das Pilottonsignal deutlich kleiner ausgelegt werden als die Antennenspule 51 für das Magnetresonanzsignal. Die sich hierdurch ergebende Verminderung der Empfindlichkeit kann durch eine entsprechende Amplitudenanhebung des Pilottons kompensiert werden.

In der Ausführungsform der Fig. 3 ist nur eine Verstimmvorrichtung 52 jeweils für die Antennenspule 51 vorgesehen, die das Magnetresonanzsignal empfangen soll. Die Antennenspule 51 für das Pilottonsignal hingegen kann permanent auf die wesentlich höhere Frequenz des Pilottonsignals abgestimmt sein, sodass sie von dem Anregungspuls nur geringfügig und ungefährlich anregt. Da die Signale bereits physisch getrennt sind, kann auch auf die Signaltrennung durch einen Diplexer 54 verzichtet werden. Die weitere Verarbeitung der Signale erfolgt wie in Fig. 3 dargestellt.

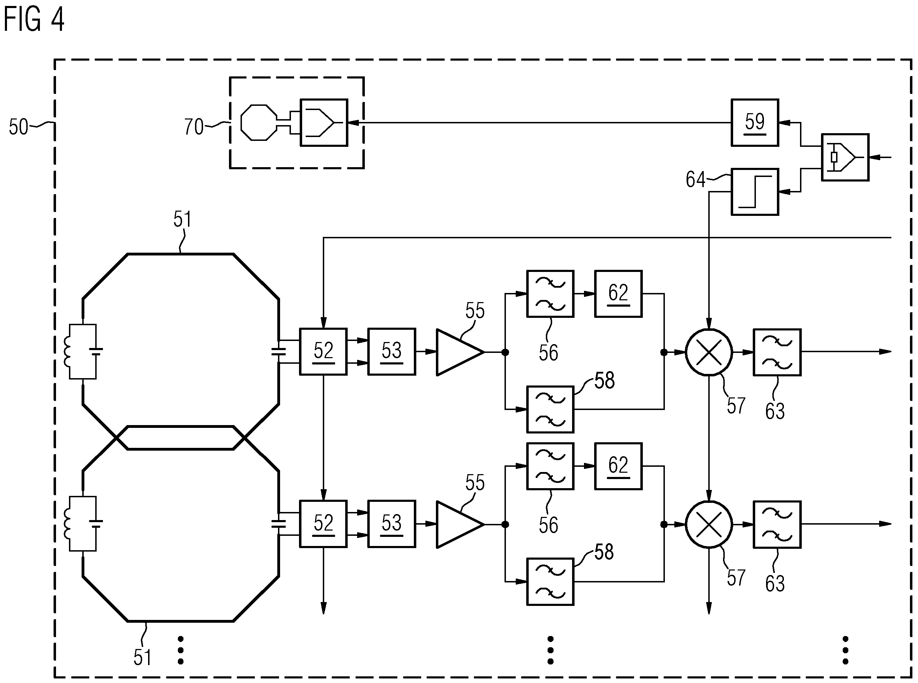

Fig. 4 zeigt eine schematische Darstellung einer beispielhaften Ausführungsform einer erfindungsgemäßen Lokalspule 50. Die Lokalspule 50 der Fig. 4 unterschiedet sich durch die Art der Mischer 57 und die Aufbereitung von Pilottonsignal und Mischerfrequenz von der Ausführungsform der Fig. 2.

Die in der Fig. 4 vorgesehenen Mischer 57 sind Schalt-Mischer, d.h. ein zu mischendes Signal wird nicht analog mit einem vorzugsweise sinusförmigen Mischsignal multipliziert, sondern mit einer Mischerfrequenz digital in der Polarität umgeschaltet bzw. ein- und ausgeschaltet. Dies entspricht aufgrund der Fourierzerlegung einem Mischen der zu mischenden Signale mit einem analogen bzw. multiplikativen Mischer mit einem Bündel an Sinussignalen mit Mischerfrequenzen mit ungeradzahligen Harmonischen des ursprünglichen digitalen Mischersignals.

Wie in Fig. 2 werden zunächst Magnetresonanzsignal und Pilottonsignal mit einer doppel-resonanten Antennenspule 51 aufgenommen. Detuning durch die Verstimmvorrichtung 52, Matching mit der Anpasschaltung 53 und Vorverstärkung durch den LNA 55 erfolgen für beide Signale gemeinsam. Anschließend erfolgt eine Trennung der Signale durch den ersten Filter 56 und den zweiten Filter 58 anhand ihrer Frequenz. Das Pilottonsignal kann durch ein Dämpfungsglied 62 in der Amplitude angepasst zu werden, um wie vorgehend beschrieben, ein optimales SNR für das Magnetresonanzsignal sicherzustellen. Der erste Filter 56 und der zweite Filter 58 stellen darüber hinaus sicher, dass keine unerwünschten Nebenfrequenzen enthalten sind, die im nachfolgenden Mischer zu störenden Mischprodukten mit störenden Frequenzen entstehen.

Der Mischer 57 als Schalt-Mischer führt dazu, dass die Eingangssignale gleichzeitig mit unterschiedlichen Frequenzen gemischt werden und entsprechend eine Vielzahl an Mischprodukten entstehen. Aus diesen werden entsprechen des nachfolgend erläuterten Frequenzschema zu Fig. 4 mit einem dritten Filter 63 Mischprodukte des Pilottonsignals und des Magnetresonanzsignals selektiert, die in einem gemeinsamen Frequenzbereich liegen und von dem nachfolgenden Empfänger 40 gemeinsam ausgewertet werden können.

In der Ausführungsform der Fig. 4 liegt das Magnetresonanzsignal beispielsweise unverändert in einem Frequenzbereich um 23,6 MHz. Für das Oszillatorsignal ist in diesem Beispiel eine Frequenz von 35 MHz vorgesehen. Aus diesem wird durch einen Signalformer 64, beispielsweise einem Schmitt-Trigger, als Mischerfrequenz ein Rechtecksignal mit 35 MHz für den Schalt-Mischer erzeugt. Ein erster Frequenzvervielfacher 59, hier ein Synthesizer, erzeugt aus dem Oszillatorsignal ein Pilottonsignal mit 117,5 MHz. Denkbar wäre eine PLL mit 117.5MHz VCXO. Die Vergleichsfrequenz wird zu 2.5MHz gewählt. Hierfür muss das VCXO-Signal durch 47 und das 35MHz ReferenzSignal durch 14 geteilt werden.

Der Schalt-Mischer, angesteuert mit einer Mischerfrequenz von 35 MHz, erzeugt zum einen mit dem Magnetresonanzsignal von 23,6 MHz ein Signal von 35 MHz - 23,6 MHz = 11,4 MHz.

Beim Pilotton sei die dritte Harmonische der Frequenz des Mischsignals betrachtet: 117,5 MHz - 3*35 MHz = 12,5 MHz.

Nach einem dritten Filter, der unerwünschte andere Mischprodukte außerhalb des gemeinsamen Frequenzbereichs von z.B. 11 MHz bis 13 MHz liegen, wird das Signalgemisch dem Empfänger 40 zur weiteren Verarbeitung zugeführt.

Neben der reduzierten Anzahl an Mischern, die darüber hinaus noch als Schalt-Mischer einfacher zu realisieren sind, wird auf vorteilhafte Weise gleichzeitig eine Umsetzung auf eine niedrigere Zwischenfrequenz vorgenommen, die die Dämpfung auf der Übertragungsstrecke reduziert und darüber hinaus das primäre Magnetresonanzsignal im Frequenzraum von dem Ausgangssignal entkoppelt.

Eine nachgeschaltete Umsetzung auf eine Zwischenfrequenz oder auch mehrere Zwischenfrequenzen für ein Frequenzmultiplex ist natürlich auch bei den Ausführungsformen der Figuren 2 und 3 denkbar.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Lokalspule für einen Magnetresonanztomographen (1), wobei die Lokalspule (50) ausgebildet ist, ein Magnetresonanzsignal und ein Pilottonsignal aufzunehmen und an einen Empfänger (40) des Magnetresonanztomographen (1) zur Auswertung weiterzuleiten, wobei das Pilottonsignal in einem ersten Frequenzbereich liegt, der disjunkt ist und einen wesentlichen Frequenzabstand zu einem zweiten Frequenzbereich des Magnetresonanzsignals aufweist und die Lokalspule (50) zumindest einen Frequenzumsetzer aufweist, der ausgebildet ist, das Pilottonsignal und das Magnetresonanzsignal in einen gemeinsamen Frequenzbereich umzusetzen.

2. Lokalspule nach Anspruch 1, wobei die Lokalspule (50) einen Eingang für ein Oszillatorsignal aufweist und die Lokalspule (50) ausgebildet ist, aus dem Oszillatorsignal einen Pilotton zum Aussenden und ein Mischersignal zum Frequenzumsetzen des Pilottonsignals mit dem Frequenzumsetzer zu erzeugen.

3. Lokalspule nach Anspruch 2, wobei die Lokalspule (50) einen ersten Frequenzvervielfacher (59) mit einem ersten Vervielfachungsfaktor zum Erzeugen des Pilottons aus dem Oszillatorsignal und einen zweiten Frequenzvervielfacher (60) mit einem zweiten Vervielfachungsfaktor zum Erzeugen des Mischersignals aus dem Oszillatorsignal aufweist,
wobei sich der erste Vervielfachungsfaktor und der zweite Vervielfachungsfaktor unterscheiden.

4. Lokalspule nach Anspruch 3, wobei sich der erste Vervielfachungsfaktor und der zweite Vervielfachungsfaktor um die Zahl 1 unterscheiden.

5. Lokalspule nach Anspruch 2, wobei das Mischersignal die gleiche Frequenz wie das Oszillatorsignal aufweist und der Frequenzumsetzer ein Schalt-Mischer ist.

6. Lokalspule nach einem der vorhergehenden Ansprüche, wobei die Lokalspule (50) eine Antennenspule (51) zum Empfang des Pilottonsignals und des Magnetresonanzsignal aufweist, wobei die Antennenspule (51) gleichzeitig eine Resonanz in dem ersten Frequenzbereich und dem zweiten Frequenzbereich aufweist.

7. Lokalspule nach einem der vorhergehenden Ansprüche, wobei die Lokalspule (50) ein Dämpfungsglied (62) aufweist, das ausgebildet ist, das Pilottonsignal auf einen Pegel vergleichbar dem oder kleiner als das zu empfangenden Magnetresonanzsignal zu dämpfen, bevor es an den Magnetresonanztomographen (1) zur gemeinsamen Auswertung mit dem Magnetresonanzsignal weitergeleitet wird.

8. Lokalspule nach einem der Ansprüche 2 bis 7, wobei die Lokalspule (50) ausgebildet ist, den Pilotton mit einem Pegel auszusenden, der zu einem Pegel des empfangenen Pilottonsignals führt, der höher ist als das zu empfangende Magnetresonanzsignal.

9. Lokalspule nach Anspruch 7 oder 8, wobei die Lokalspule (50) eine Mehrzahl an Antennenspulen (51) und eine Mehrzahl an den Antennenspulen (51) zugeordneten Dämpfungsgliedern (62) aufweist, wobei sich ein Dämpfungswert für mindestens zwei Dämpfungsglieder unterscheidet.

10. Magnetresonanztomograph mit einer Lokalspule (50) nach einem der vorhergehenden Ansprüche, einem Oszillator und einem Empfänger (40), wobei der Magnetresonanztomograph (1) ausgebildet ist, mit dem Oszillator der Lokalspule (50) das Oszillatorsignal bereitzustellen und mit dem Empfänger (40) sowohl das Magnetresonanzsignal als auch das Pilottonsignal auszuwerten.
